# EUROPEAN PATENT APPLICATION

(11) **EP 4 711 439 A1**
(43) Date of publication of application: **18.03.2026**
(21) Application number: 24200633.6
(22) Date of filing: 16.09.2024
(51) Int. Cl.: C12M 1/00, C12M 1/34, C12M 1/36

(54) **FLOW SYSTEM WITH RECIRCULATION LOOP**

(71) Applicant: Sartorius Stedim Biotech GmbH, 37079 Göttingen (DE)
(72) Inventor: Steinwedel, Tobias, 37079 Göttingen (DE); Klemm, Anna, 37079 Göttingen (DE); Sachmann, Tobias, 34302 Guxhagen (DE); Kühnel, Christian, 37079 Göttingen (DE); Scheck, Lara, 37079 Göttingen (DE)
(74) Representative: Cohausz & Florack

(57) **Abstract**

The present invention relates to a flow system for the continuous treatment of a process liquid. The flow system comprises a recirculation loop comprising a compensation tank having an inlet and an outlet, a circulation line connecting the inlet and outlet of the compensation tank to one another, at least one sensor for determining at least one liquid parameter, supply lines for the process liquid and at least one treatment liquid which are connected to the recirculation loop, and discharge lines for treated process liquid and waste streams connected to the recirculation loop.

## Description

The present invention relates to a flow system for the continuous treatment of a process liquid. The present invention further relates to a device comprising the flow system. The present invention further relates to a use of said device for the treatment of a process liquid.

It is known to subject process liquids to certain treatments as part of biotechnological processes, for example for adjusting the pH of a given process liquid or for inactivating viruses in a process liquid. These processes involve mixing the process liquid with a treatment liquid, which causes a temporary change in the physical or chemical properties of the process liquid, for example, or starts a reaction within the process liquid. Often, such mixtures of process liquid and treatment liquid must be incubated for a certain amount of time before they may be processed further.

In conventional devices, the above-described process steps are often carried out sequentially, for example in a tank. These so-called "batch processes" do not run continuously, which is unfavorable because of long holding times between subsequent process steps. Continuous processes, in contrast thereto, can significantly shorten the time the product remains in the bioprocess, thus making it possible to carry out the individual work steps of the bioprocess on a much smaller scale, which can save on consumables and space requirements.

A core difficulty of continuous processes is achieving to stay within narrow working windows many bioprocesses require. For example, in case of inactivating viruses in a process liquid by adjusting the pH, a maximum deviation of ± 0.1 pH units is allowed. If this allowable range is exceeded, the potentially contained viruses cannot be sufficiently inactivated and/or the process liquid is adversely affected. As a result, the process must be interrupted and the process liquid discarded. Moreover, any interruption to the process may have further adverse implications for a larger bioprocess due to coordinated flow rates between the individual systems within a connected process chain.

Currently available systems that carry out continuous inline treatment of a process liquid usually feed a treatment liquid (acid, base or salt solution, for example) directly into the process liquid line via a T-piece. Either fixed volume ratios or feedback control based on a specific sensor value (pH, conductivity, etc.) are used.

For example, in the case of feedback control, a deviation from the allowable range can occur, particularly after external faults. In this case, the incorrectly treated process liquid must be led out of the system and then discarded or readjusted. In the case of virus inactivation by adjusting the pH of a process liquid, the detection of deviations in the pH value is particularly important, as the pH value is a critical process parameter. The comparatively long response time of electrochemical pH electrodes makes timely detection difficult and there is therefore a risk that the parameters for successful virus inactivation will not be met at all times. This problem is particularly present in flow systems where the pH is measured immediately downstream of the point where process and treatment liquids are added, because the liquid's dwell time within a pH flow-through cell is generally many times shorter than the response time of electrochemical pH sensors. Short-term deviations of the pH value from the allowable range can therefore not be reliably detected. Even with successful detection, readjustment of the pH value is not easily possible, as this requires further addition points for acid or base or a further line to return wrongly treated product to an earlier point of entry.

Against this background, it is an object of the present invention to overcome the drawbacks in the prior art. In particular, it is an object of the present invention to provide an improved device and/or flow system for treating a process liquid in a continuous process. Preferably, the device and/or flow system according to the invention shall allow precise process control, a more stable measurement of a liquid parameter, readjustment in case a liquid parameter falls outside an allowable range, and/or improved versatility compared to conventional devices and flow systems.

The object named above is solved in accordance with the present invention by a flow system for the continuous treatment of a process liquid, comprising:
- a recirculation loop comprising a compensation tank having an inlet and an outlet and a circulation line connecting the inlet and outlet of the compensation tank to one another,
   wherein the circulation line comprises a pump for generating a circulation flow,
- at least one sensor for determining at least one liquid parameter,
- supply lines for the process liquid and at least one treatment liquid which are connected to the recirculation loop, and
- discharge lines for treated process liquid and waste streams connected to the recirculation loop.

In the context of the present disclosure, a process liquid is intended to be understood as a liquid that is subjected to a treatment or modification process to alter its chemical or physical properties, for example its pH value or its viral load.

In the context of the present disclosure, a continuous treatment or continuous process is understood to be a process where the input materials, especially the process liquid and treatment liquid(s), and the output products flow through a system in a sustained manner over time, allowing for the steady production of an output product over an extended period. For example, a continuous process may be designed to operate with minimal interruptions, including scenarios where there are intermittent breaks in the generation or supply of input materials, as long as the overall operation is maintained without complete shutdowns, and the process may be capable of resuming seamlessly after such breaks.

Compared to batch processes, continuous processes have the further advantage that sequential process steps may be conducted in parallel, which potentially increases the throughput of the process. For example, in a process comprising (1) acidifying a process liquid, (2) incubating the acidified process liquid for a predetermined amount of time, and (3) neutralizing the incubated process liquid, a batch process requires that all steps are carried out sequentially (e.g., in a single tank), while a continuous process may conduct all steps in parallel in different parts of the production line.

In the context of the present disclosure, a circulation flow is intended to be understood as a movement of liquid, which may be a continuous movement, through the recirculation loop. It is generally preferred that the circulation flow is generated by a pump, also referred to as "recirculation pump". For example, the liquid may be drawn through an outlet of the compensation tank, transported through the circulation line, and returned to the compensation tank via an inlet. The circulation flow may be characterized by one or more of the following parameters: flow rate (i.e., the volume of liquid flowing per unit of time, typically expressed in units such as liters per minute [L/min]), flow direction (i.e., the orientation or path along which the liquid flows, which may be specified as forward or reverse), velocity (i.e., the speed at which the liquid flows through the system, often expressed in meters per second [m/s]).

In the context of the present disclosure, the flow rate of a liquid may be controlled or regulated by a pump. A positive flow rate indicates forward flow, e.g. caused by forward pumping, while a negative flow rate indicates a reverse flow, e.g. caused by reverse pumping. A flow rate of zero indicates standstill of the liquid, e.g. caused by stopping the pump.

In the context of the present disclosure, the term "upstream" is intended to be understood as the direction or location in a flow system that is closer to the source or point of entry of the liquid, such that components positioned upstream are encountered earlier in the flow path. In the context of the recirculation loop, the term "upstream" is intended to be understood as the direction or location opposite to the circulation flow of the liquid.

In the context of the present disclosure, the term "downstream" is intended to be understood as the direction or location in a flow system that is farther from the source or point of entry of the liquid. Components positioned downstream are encountered later in the flow path relative to upstream components. In the context of the recirculation loop, the term "downstream" is intended to be understood as the direction or location in the same direction as the circulation flow.

In the context of the present disclosure, a treatment liquid is intended to be understood as a liquid introduced into a process to achieve a specific modification or adjustment of the process liquid, such as altering the pH, neutralizing contaminants, or adding desired chemical species to achieve the desired characteristics in the process liquid. A treatment liquid may comprise an acidic or basic compound. A treatment liquid may comprise a buffering compound, a salt, and/or a solvent.

In the context of the present disclosure, the term "liquid parameter" is intended to be understood as a measurable value indicative of a physical or chemical property of a liquid. For example, the liquid parameter may be measurable with a sensor such as a flow cell. For example, the liquid parameter may be selected from the group consisting of pH, conductivity, optical density / absorbance, redox potential, turbidity, refractive index, total organic carbon (TOC), chemical oxygen demand (COD), viscosity, particle size distribution, inelastic light scattering (also known as Raman spectroscopy), fluorescence, total solid mass, total solid volume, viable cell concentration, total cell concentration, light scattering (also known as optical density), density, and temperature.

In the context of the present disclosure, the term "sensor" is intended to be understood as a device or component that is designed to measure or detect the at least one liquid parameter of a liquid stream passing through it. For example, the sensor may be in form of a sensor or detector which is arranged at least partially inside the liquid stream, or may be in form of a flow cell. For example, the sensor may be positioned within the recirculation loop to provide real-time data on the liquid parameter, ensuring optimal control and monitoring of the circulating liquid. For example, the data collected by the flow measuring cell may be used for process control.

In the context of the present disclosure, the term "connected to the recirculation loop" is meant to be understood as having a fluid-tight connection between the additional components of the present invention (such as the supply and discharge lines) and the recirculation loop (i.e., the circulation line or the compensation tank). Such a connection may be open or may be closable, for example by a valve.

Due to the compensation tank, the recirculation loop according to the invention has an increased retention time of liquid and an increased inertia compared to known systems, which is advantageous for generating a uniform product, i.e. treated process liquid.

The object named above is further solved in accordance with the present invention by a device for the continuous treatment of a process liquid, comprising a flow system according to the invention, in particular a recirculation loop.

For example, the device may additionally comprise at least one reservoir in which a respective treatment liquid is stored and from which it can be supplied to the flow system.

The object named above is further solved in accordance with the present invention by a use of a device according to the invention for adjusting the pH of the process liquid, for inactivating viruses in the process liquid, for solvent/detergent inactivation, for buffer conditioning, for transfection or transformation of cells, for plasmid production, for lysis of cells, for production of viral vectors, for carrying out a bio-conjugation reaction, for in vitro transcription, or for vesicle encapsulation. For example, the device may be used for treating a cell suspension, e.g. by acidifying, to precipitate DNA and cell fragments, e.g. prior to a filtration step. For example, the device may be used to add an excipient to a composition comprising an active compound.

Various embodiments of the flow system, the device and the use are described in the following. The individual embodiments are in each case individually applicable to the flow system, the device and the use. The individual embodiments may furthermore be combined with each other at will.

In a preferred embodiment, the compensation tank is arranged along the circulation flow between the supply lines and the sensor.

In this context, the term "between the supply lines and the sensor" is intended to be understood as a location between the point where the supply lines open into the circulation line and the point at which the at least one sensor is arranged. Thus, the process liquid and treatment liquid(s), after being fed into the recirculation loop, must pass through the compensation tank before the at least one liquid parameter is determined.

In this embodiment, the compensation tank serves as an additional mixing chamber for the process liquid and treatment liquid(s) before the at least one liquid parameter of the mixture is measured, thus increasing the inertia, which contributes to an improved stability of the liquid parameter(s) when measured at the sensor, and an improved robustness of the treatment process. Without wishing to be bound by theory, it is currently assumed that due to the increased retention time and mixing volume of the process liquid and treatment liquid(s), short-term control deviations are dampened and at the same time, the overall rate of change of the liquid parameter decreases. As a result, the at least one liquid parameter (e.g., the pH value) of the treated process liquid may be determined more reliably and more precisely.

In an exemplary embodiment, the supply lines for the process liquid and the treatment liquid are connected to the recirculation loop at spaced-apart locations. In another exemplary embodiment independent therefrom, the discharge lines for treated process liquid and waste streams are connected to the recirculation loop at spaced-apart locations.

In this context, the term "connected to the recirculation loop at spaced-apart locations" is intended to be understood as having separate connection ports at the recirculation loop which are spaced apart from one another. For example, in this embodiment, the supply line for the process liquid opens directly into the recirculation loop (e.g., into the compensation tank or, preferably, into the circulation line) at a first connection point, and the supply line(s) for the at least one treatment liquid opens directly into the recirculation loop at one or more second connection point(s) which is/are spaced apart from the first connection point. In particular, the process liquid and treatment liquid(s) are not mixed prior to entering the recirculation loop.

For example, in case that the process liquid and treatment liquid(s) may differ significantly with respect to the at least one treatment parameter, this may lead to adverse effects if process and treatment liquids are directly mixed prior to entering the recirculation loop, such as unintended warming or high local concentrations of the treatment liquid. In this exemplary embodiment, the process and treatment liquids are admixed into the circulation flow which comprises a mixture of process and treatment liquids having an intermediate liquid parameter. Thereby, the recirculation loop has the advantage that it allows for gentler mixing of the process and treatment liquids, thereby avoiding certain adverse effects. The aforementioned advantages are particularly pronounced when the process and treatment liquids enter the recirculation loop via the circulation line, because of the increased turbulence associated therewith.

For example, if the discharge lines for treated process liquid and waste streams are connected to the recirculation loop at separate locations, off-specification product can be directly and efficiently discarded without passing through any shared pathways, thereby eliminating dead volume and reducing the risk of cross-contamination.

In an exemplary embodiment, the supply and discharge lines are setup so that their respective flow rates can be controlled independently of one another, e.g. by at least one PID controller.

In the context of the present disclosure, the term "control" is intended to be understood as a mechanism of controlling and/or regulating. For example, proportional-integral-derivative (PID) controllers are known for their high precision and stable regulation capabilities.

In the context of the present disclosure, the flow rate of a liquid may be controlled or regulated by acting on a pump and/or valve. A positive flow rate indicates forward flow, e.g. caused by forward pumping, while a negative flow rate indicates a reverse flow, e.g. caused by reverse pumping. A flow rate of zero indicates standstill of the liquid, e.g. caused by stopping the pump.

In this exemplary embodiment, the flow system has the advantage that it allows for precise control of the liquid flow.

In an exemplary embodiment, the discharge line for treated process liquid is connected to the recirculation loop by means of a void-free valve.

In the context of the present disclosure, a void-free valve is a type of valve engineered to minimize or completely eliminate dead space within its internal structure. Dead space refers to areas within the valve where liquid or gas can stagnate, leading to potential contamination, unwanted mixing, or degradation of the fluid. In a void-free valve, the internal geometry is designed so that, when the valve is in any position (open, closed, or partially open), there are no cavities, pockets, or spaces where fluid can be trapped or remain stationary. Especially in the context of a continuous process, it has shown to be highly advantageous to avoid contaminations within the flow system. Using a void-free valve for the connection between the recirculation loop and the discharge line for treated process liquid contributes to a reduction of unwanted contaminations.

Exemplary void-free valves include diaphragm valves.

In an exemplary embodiment, the circulation flow has a flow rate which is at least 10 times the flow rate in the supply lines.

In general, a difference in flow rate results in the formation of turbulence in the circulation line and in the compensation tank, which causes the process and treatment liquids to mix. The research leading up to the invention has surprisingly found that a flow rate difference of at least 10-fold ensures homogenous mixing not only in the circulation line but also within the compensation tank, such that, for example, an active mixer is not necessary. A recirculation loop without an active mixer is advantageous because it requires less maintenance.

The flow rate of the circulation flow may be at least 10 times, at least 20 times, or at least 30 times higher than the flow rate in the supply lines, e.g. up to 100 times higher than the flow rate in the supply line.

In an exemplary embodiment, the compensation tank contains a variable amount of liquid.

In the context of the present disclosure, the phrase "the compensation tank contains a variable amount of liquid" is intended to be understood in that either the compensation tank has a constant inner volume and a variable fill level, or the compensation tank has a variable inner volume, e.g. in form of a flexible bag.

It is preferred that the compensation tank is a flexible bag, as this reduces exposure to gas and foaming of the liquid compared to a solid tank - although some foaming may occur regardless. Without wishing to be bound to theory, it is assumed that a flexible bag minimizes the area of air-liquid interface and has no headspace, thus reducing foaming. Moreover, a flexible bag may provide gentler fluid dynamics than a solid tank, reducing turbulence and thus reducing foaming.

If the liquid parameter is determined to be outside the allowable range, the flow system of the invention allows a wide range of reaction options for readjustment. For example, the process liquid may be readjusted within the recirculation loop by adding further process liquid or treatment liquid(s) while leaving the connection to the discharge lines closed. In this embodiment, the compensation tank, due to its variable volume, is set up to collect the additional volume added. As a result, preferably no product has to be discarded and the product yield is increased.

For example, in case of pH adjustment of a process liquid, if the pH value falls outside the allowable range due to excessive addition of treatment liquid, additional process liquid is introduced into the recirculation loop to bring the pH value back into the allowable range. A dedicated line with a pump for adding a neutralizing agent thus becomes obsolete.

For example, if the volume of the compensation tank in the recirculation loop is not sufficient to absorb the additional volume of liquid required for readjustment, the excess volume can be discharged via the waste line. Only as much product is discharged as is necessary to avoid overfilling the compensation tank. Thereby, the flow system according to the invention has the advantage of reducing waste production and keeping product loss at a minimum.

In an exemplary embodiment, the compensation tank is set up so that the amount of liquid in it is continuously measured and the flow system is set up to regulate the circulation flow rate depending on the amount of liquid in the compensation tank.

In the context of the present disclosure, the term "regulate" shall be understood as maintaining a variable within a desired or allowable range or set of limits by automatically adjusting system inputs or outputs based on a feedback mechanism. For example, the variable can be a flow rate of a liquid. For example, the feedback mechanism may be based on measurements of a liquid parameter such as the pH value, and/or on a flow rate of a liquid.

In the context of the present invention, the term "allowable range" is intended to be understood as the specified range of values of the at least one liquid parameter within which a pH adjustment process or reaction must be maintained to ensure optimal performance, safety, and compliance with predefined criteria. For example, the allowable range may be determined based on the requirements of the process, including the desired chemical reactions, biological activity, or product specifications. For example, the allowable range may define the acceptable limits around a setpoint value for the liquid parameter(s), beyond which the process may become ineffective, unsafe, and/or result in the production of off-spec material.

For example, the amount of liquid present in the compensation tank may be measured by weighing, or may e.g. be measured by a float sensor or any other suitable sensor.

For example, the recirculation flow rate may be regulated at least partially based on the fill level of the compensation tank while treating a liquid in order to ensure homogeneous mixing while reducing or avoiding foaming for each fill level.

In an exemplary embodiment, the compensation tank is mounted in a mass-decoupled manner.

In the context of the present disclosure, the term "mass-decoupled" refers to the design and mounting of a component, such as a tank in a flow system, in a manner that isolates it from external vibrations, forces, or movements. Thereby, the weight of the tank may be measured independently of any dynamic forces or mechanical interactions from connected lines, equipment, or structural elements.

For example, the compensation tank may be weighed to detect the fill level. A special geometry of the line connections may decouple the weighing and thus minimize the influence of the connected lines on the weighing signal. For example, angled connectors may be used to align the lines horizontally so that the compensation tank can move vertically without increased force. By minimizing or eliminating the influence of external factors, the weight of the tank can be accurately determined through weighing. This, in turn, provides for improved process control when using the flow system according to the invention.

In an exemplary embodiment, the recirculation loop further comprises a control unit which is set up to continuously receive measurement data representative of the at least one liquid parameter and to continuously receive measurement data representative of the amount of liquid in the compensation tank, and which is further set up to continuously control the flow rates in the supply and discharge lines depending on the received measurement data.

For example, the control unit may directly or indirectly communicate with the at least one sensor to receive, repeatedly or continuously, measurement data of the at least one liquid parameter. The control unit may have means to calculate, based on the at least one liquid parameter, a rotational speed of at least one pump arranged in one of the supply lines or discharge lines, and may directly or indirectly act on said pump accordingly to regulate the flow of liquids in the flow system. Moreover, the control unit may have means to act on at least one valve in order to further regulate the flow of liquids in the flow system, e.g. by opening or closing said valve.

In an exemplary embodiment, the treatment liquid is acid and the recirculation loop does not comprise a supply line for base, or the treatment liquid is base and the recirculation loop does not comprise a supply line for acid, wherein the liquid parameter is the pH value.

In the context of the present disclosure, the term "acid" is intended to mean a liquid, for example an aqueous solution, comprising at least one acidic compound. Acidic compounds are chemical compounds that can donate a proton (H⁺ ion) to another substance and/or that can accept an electron pair. Acidic compounds typically have a pH less than 7 in aqueous solutions. Exemplary acidic compounds include, but are not limited to, Hydrochloric Acid, Sulfuric Acid, Acetic Acid, Citric Acid, Phosphoric Acid, Nitric Acid, Lactic Acid, Formic Acid, Tartaric Acid, Malic Acid, Glycine, and partially deprotonated derivatives thereof.

In the context of the present disclosure, the term "base" is intended to mean a liquid, for example an aqueous solution, comprising at least one basic/alkaline compound. Basic compounds are chemical compounds that can accept a proton (H⁺ ion) from another substance and/or that can donate an electron pair. Basic compounds typically have a pH greater than 7 in aqueous solutions. Exemplary basic compounds include, but are not limited to, Sodium Hydroxide, Potassium Hydroxide, Ammonium Hydroxide, Calcium Hydroxide, Potassium Carbonate, Magnesium Hydroxide, Sodium Carbonate, Sodium Phosphate, tris(hydroxymethyl)aminomethane (Tris), and differently protonated derivatives thereof, and derivatives thereof having different counter ions.

The acid/base can be provided in any suitable buffer and concentration known in the field. A person skilled in the art is aware of common interactions between an acid or base and the process liquid to be treated, and will choose a suitable acid or base accordingly.

For example, if the pH value falls outside the allowable range due to excessive addition of acid/base, additional process liquid is introduced into the recirculation loop to bring the pH value back into the allowable range. A dedicated line with pump for adding neutralizing liquid (i.e.: base/acid) thus becomes obsolete.

In an exemplary embodiment, the device further comprises a flow path arranged to receive treated process liquid, wherein the flow path is configured such that the passage of the treated process liquid through the flow path requires a predetermined minimum period of time.

Precise incubation times are often part of biotechnological processes, such as in the case of viral inactivation, where a process liquid may be brought to a predefined pH value, may then be kept at said pH value for a certain incubation time, and may then be further processed. For example, in such a setting, a flow path may allow for precise treatment of the process liquid by ensuring that the incubation time of the pH-adjusted liquid is complied with.

For example, the flow path may be connected to the discharge line for treated process liquid, i.e., downstream of the recirculation loop. For example, the flow path may open into another component of the device, such as a further recirculation loop.

In an exemplary embodiment, the device further comprises a surge tank. Therein, the surge tank may be set up to receive an incoming process material. Therein, an inlet of the surge tank is connected to an outlet of an upstream unit operation and an outlet of the surge tank is connected to the supply line for process liquid.

In this context, a process material may be understood as a material originating from an earlier step of the bioprocess. For example, the process material may be an output material from a chromatography unit, filtration unit or extraction unit, or from an earlier step of chemical synthesis, for example.

Preferably, the inlet of the surge tank is connected to an outlet of a chromatography system. Bind-elute chromatography systems in particular are inherently incompatible with a continuous process because the process material, which is part of an eluate of the chromatography process, is provided in a non-continuous manner, i.e. in batches or the like. Moreover, chromatography systems in general are known to provide process material in a non-uniform concentration. However, when using a surge tank according to the present embodiment, these incompatibilities may be overcome by, for example, adding additional substance(s) and/or blending.

Optionally, the surge tank is set up to blend the incoming process material. Therein, the surge tank may comprise an active mixer such as a propeller or paddle mixer.

Optionally, the surge tank is set up to receive a buffer. Therein, a buffer is usually supplied to the process material in order to adjust its pH value, salt concentration, and/or volume (i.e., concentration of a substance of interest). The buffer may be in the form of a liquid, a salt or another solid. A person skilled in the art is aware of common interactions between a buffer and the process material, and will choose a suitable buffer accordingly.

Preferably, the surge tank is set up to provide an essentially continuous outflowing liquid stream. Therein, the surge tank may comprise means for determining a fill level of the surge tank. Moreover, the surge tank may comprise means to avoid overflow and running dry, e.g. by having a suitable size and/or by regulating the fill level of the surge tank by providing additional volumes of a liquid, if necessary. Alternatively or additionally, the outlet of the surge tank may comprise a valve or pump which is regulated based on the fill level of the surge tank.

The surge tank may comprise means for determining at least one liquid parameter, such as a sensor for determining the pH value, for example. Therein, the surge tank and/or the sensor may communicate with the flow system to regulate the flow of liquids based on the at least one liquid parameter determined in the surge tank. Further features and advantages of the recirculation loop and the device and the use according to the present invention emerge from the following description of exemplary embodiments where reference is made to the attached Figures.

In the figures,
- Fig. 1: shows an exemplary embodiment of the flow system of the invention,
- Fig. 2: shows an exemplary embodiment of a device according to the invention, and
- Fig. 3: shows another exemplary embodiment of a device of the invention.

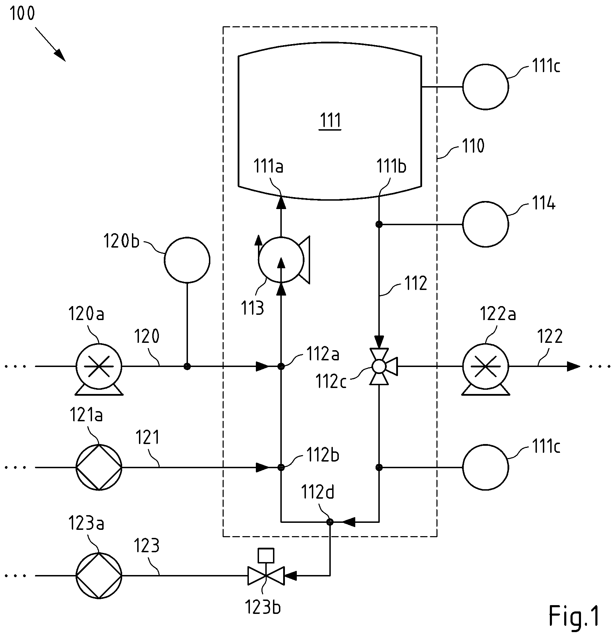

Fig. 1 shows a flow system 100 for the continuous treatment of a process liquid. The flow system 100 comprises a recirculation loop 110 comprising a compensation tank 111 and a circulation line 112. The compensation tank 111 has an inlet 111a and an outlet 111b. The circulation line 112 connects the inlet 111a and the outlet 111b. The circulation line 112 further comprises a recirculation pump 113 which is configured to generate a circulation flow (indicated in Fig. 1 by arrows along the circulation line 112).

The flow system 100 further comprises a sensor 114 for determining at least one liquid parameter, such as the pH value or the conductivity of a liquid flowing through the recirculation loop 110. According to the embodiment of Fig. 1, the sensor 114 is designed as a flow cell. Additionally, the flow system 100 further comprises a sensor 115 for measuring the flow rate of the liquid in the circulation line 112.

The flow system 100 further comprises supply lines 120, 121 for supplying a process liquid and a treatment liquid, respectively, to the recirculation loop 110. The process liquid supply line 120 comprises a pump 120a designed to pump process liquid. Similarly, the treatment liquid line 121 comprises a pump 121a designed to pump treatment liquid. Moreover, the supply line 120 for process liquid comprises flow rate sensor 120b for measuring the flow rate of the process liquid.

The flow system 100 further comprises discharge lines 122, 123 for removing treated process liquid and waste streams, respectively. Discharge line 122 for treated process liquid comprises a pump 122a, and discharge line 123 for waste streams comprises pump 123a and may comprise a valve 123b. It is generally appreciated that the valve 123b may be arranged in the circulation line 112 instead of in the discharge line 123 as shown.

The supply and discharge lines 120-123 are connected to the recirculation loop 110 by means of ports 112a-d in the circulation line 112, each port 112a-d facilitating a connection with one of the supply and discharge lines 120-123. Some of the ports 112a-d may comprise a valve, as seen e.g. in Fig. 1 for the connection between the circulation line 112 and the treated process liquid discharge line 122, which are connected by port 112c comprising a valve. According to Fig. 1, the ports 112a-d are located at spaced-apart locations such that the supply and discharge lines 120-123 are all connected to the recirculation loop 110 at spaced-apart locations.

The compensation tank according to Fig. 1 is arranged along the circulation flow between the supply lines 120, 121 and the sensor 114. Thus, process and treatment liquids entering the recirculation loop 110 are first admixed to the liquid stream flowing through the circulation line 112 and enter the compensation tank 111, where they are further mixed and remain for a certain period before being transported through the outlet 111b into the circulation line 112 and through the sensor 114. Thereby, the flow system 100 provides an inertia between the liquid mixing and the measurement of the liquid parameter(s).

According to Fig. 1, the compensation tank further comprises a load sensor 111c configured to determine the fill level of the compensation tank 111. For example, the load sensor 111c may be a weight scale, wherein it is preferred that the compensation tank 111 is mounted in a mass-decoupled manner. Alternatively, the load sensor 111c may be a level sensor based on radar or capacitance measurements.

It is generally appreciated that at least the pumps 113, 120a, 121a, 122a, 123a, and at least the valves 112c, 123b, preferably all pumps and valves of the flow system 100, may be controlled by a common control unit.

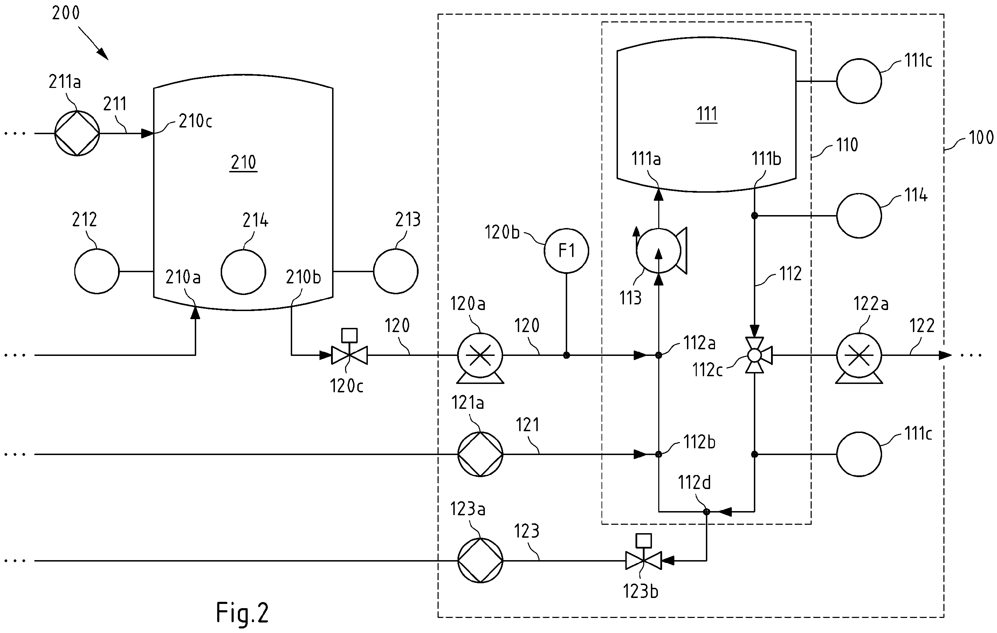

Looking to Fig. 2, an embodiment of a device 200 according to the invention is shown. The device 200 contains the flow system 100 comprising the recirculation loop 110 as described above in Fig. 1.

The device 200 further comprises a surge tank 210 having a first inlet 210a and an outlet 210b. The first inlet 210a of the surge tank 210 is connected to an upstream unit, such as a chromatography unit (not shown); the outlet 210b is connected to supply line 120, with valve 120c being arranged between the surge tank 210 and the pump 120a. Via a second inlet 210c, the surge tank 210 is connected to a supply line 211 for buffer, which according to Fig. 2 further comprises a pump 211a. Thereby, the surge tank 210 is set up to receive process material through its first inlet 210a and optionally buffer through its second inlet 210c.

The surge tank 210 further comprises sensors 212, 213 for determining at least one liquid parameter, such as the pH value, and the fill level of the surge tank 210, respectively. The sensors 212, 213 may communicate with a control unit (not shown) to regulate the flow of liquids through the flow system 100. The surge tank 210 further comprises active mixer 214 for blending the incoming process material and optionally the buffer.

In the embodiment shown in Fig. 2, the surge tank 210 is set up to provide an essentially continuous outflowing liquid stream comprising the process material and optionally the buffer. Arrows indicate the direction of liquid flow.

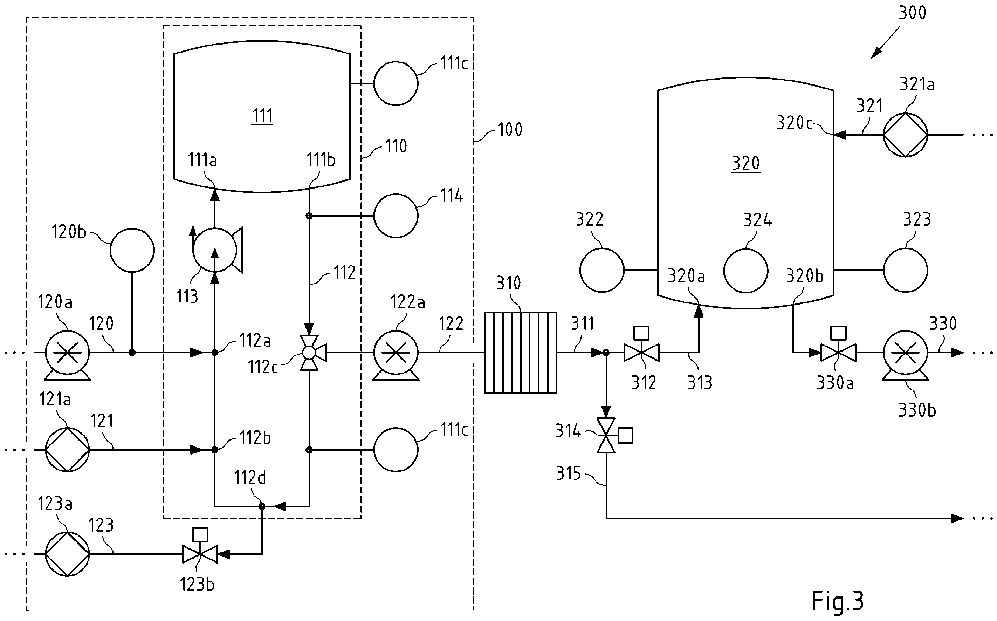

Fig. 3 shows another embodiment of a device according to the invention. The device 300 comprises a flow system 100 comprising a recirculation loop 110 according to the invention. The embodiment shown in Fig. 3 can be combined with the embodiment shown in Fig. 2.

The device 300 comprises a flow path 310 arranged downstream of the recirculation loop 110 along the discharge line for treated process liquid 122 (arrows indicate direction of flow). The flow path 310 is configured such that the passage of the liquid requires a certain amount of time in order to complete the liquid treatment.

Downstream of the flow path 310, a line 311 is arranged to transport the treated process liquid further through the device 300. According to the embodiment shown in Fig. 3, the treated process liquid passes a T-junction to either be transported through valve 312 and line 313 towards a neutralization tank 320, or to be discharged as waste through valve 314 and waste discharge line 315.

The device 300 further comprises a neutralization tank 320 having a first inlet 320a and an outlet 320b. The first inlet 320a of the neutralization tank 320 is connected to the line 313 for treated process liquid; the outlet 320b is connected to line 330 comprising a valve 330a and a pump 330b for further transport to a downstream unit. Via a second inlet 320c, the neutralization tank 320 is connected to a supply line 321 for neutralization liquid, which according to Fig. 3 further comprises a pump 321a. Thereby, the neutralization tank 320 is set up to receive treated process liquid through its first inlet 320a and a neutralization liquid through its second inlet 320c.

The neutralization tank 320 further comprises sensors 322, 323 for determining at least one liquid parameter, such as the pH value, and the fill level of the surge tank 320, respectively. The neutralization tank 320 further comprises active mixer 324 for blending the incoming treated process liquid and neutralization liquid.

The invention will now be further described with respect to the Example below, which is to be understood as merely exemplary, non-limiting embodiments of the present invention.

### Example 1: low-pH virus inactivation

A device according to an exemplary embodiment of the invention was used for the continuous inactivation of viruses in a process liquid.

A process material was continuously provided from the outlet of a chromatography unit and pumped into a surge tank, where the process material was blended by an active mixer to generate a process liquid. The pH value of the process liquid was determined by a pH sensor integrated into the surge tank. A pH value of 4.6 ± 0.2 was determined for the process liquid.

The process liquid was continuously transported through the outlet of the surge tank and the supply line to a flow system comprising a recirculation loop. The circulation line and compensation tank of the recirculation loop were filled with a mixture of process liquid and a Glycine buffer, pH 2.5, as an acid / treatment liquid. The treatment liquid entered the recirculation loop at a spaced-apart location in the circulation line of the recirculation loop. A circulation pump arranged in the circulation line generated a circulation flow of the mixture of process and treatment liquids, thus transporting it into the compensation tank. Since the flow rate in the circulation line was kept approx. 10-100 times higher than the flow rate of the process liquid in the supply line, an efficient mixing was achieved. Moreover, due to the high flow rate of the circulation flow, the liquid caused turbulence in the compensation tank sufficient for thorough mixing of the process and treatment liquids. The circulation flow rate was adjusted based on the fill level of the compensation tank to ensure that sufficient mixing was continuously accomplished.

The pH value of the mixture was measured in a pH flow cell arranged downstream of the compensation tank. A pH value of 3.6 ± 0.1 was determined, which is sufficient for virus inactivation in the process liquid and characterized a sufficient treatment of the process liquid. In fact, the adjusted pH value was found to be precisely reached under various conditions, including the initial phase after an intermittent process stop and after a change in the chromatography unit leading to an initial pH value of the process liquid of 5.2.

A void-free valve was opened towards the discharge line for treated process liquid, and the treated process liquid was further transported towards a flow path designed for a minimum retention time of 30 minutes. In passing through the flow path, the incubation time necessary for virus inactivation in the process liquid was passed, thus generating a virus-inactivated process liquid.

From the flow path, the virus-inactivated process liquid was further transported to a neutralization tank where it was neutralized with 2 M TRIS as a neutralization liquid with active mixing until a pH value of 7.0 ± 0.1 was reached, which characterizes the neutralized process liquid. The neutralized process liquid was transported further downstream in the bioprocess pipeline.

The example shows that the device and flow system according to the invention are suitable for the continuous treatment, in particular the pH adjustment, of a process liquid.

### List of reference numbers

| | | | |
|---|---|---|---|
| 100 | flow system | 210c | second inlet |
| 110 | recirculation loop | 211 | supply line for buffer |
| 111 | compensation tank | 211a | pump |
| 111a | inlet | 212 | sensor |
| 111b | outlet | 213 | sensor |
| 111c | load sensor | 214 | active mixer |
| 112 | circulation line | 300 | device |
| 112a-d | ports | 310 | flow path |
| 113 | recirculation pump | 311 | line |
| 114 | sensor | 312 | valve |
| 115 | sensor | 313 | line |
| 120 | supply line for process liquid | 314 | valve |
| 120a | pump | 315 | waste discharge line |
| 120b | flow rate sensor | 320 | neutralization tank |
| 121 | supply line for treatment liquid | 320a | first inlet |
| 121a | pump | 320b | outlet |
| 122 | discharge line for treated process liquid | 320c | second inlet |
| | | 321 | supply line for neutralization liquid |
| 122a | pump | | |
| 123 | discharge line for waste streams | 321a | pump |
| 123a | pump | 322 | sensor |
| 123b | valve | 323 | sensor |
| 200 | device | 324 | active mixer |
| 210 | surge tank | 330 | line |
| 210a | first inlet | 330a | valve |
| 210b | outlet | 330b | pump |

## Claims

1. Flow system for the continuous treatment of a process liquid, comprising:
- a recirculation loop comprising a compensation tank having an inlet and an outlet and a circulation line connecting the inlet and outlet of the compensation tank to one another,
wherein the circulation line comprises a pump for generating a circulation flow,
- at least one sensor for determining at least one liquid parameter,
- supply lines for the process liquid and at least one treatment liquid which are connected to the recirculation loop, and
- discharge lines for treated process liquid and waste streams connected to the recirculation loop.

2. Flow system according to claim 1, wherein the compensation tank is arranged along the circulation flow between the supply lines and the sensor.

3. Flow system according to any one of claims 1 or 2, wherein the supply lines for the process liquid and the treatment liquid are connected to the recirculation loop at spaced-apart locations, and/or wherein the discharge lines for treated process liquid and waste streams are connected to the recirculation loop at spaced-apart locations.

4. Flow system according to any one of the preceding claims, wherein the supply and discharge lines are set up so that their respective flow rates can be controlled independently of one another, e.g. by at least one PID controller.

5. Flow system according to any one of the preceding claims, wherein the discharge line for treated process liquid is connected to the recirculation loop by means of a void-free valve.

6. Flow system according to any one of the preceding claims, wherein the circulation flow has a flow rate which is at least 10 times the flow rate in the supply lines.

7. Flow system according to any one of the preceding claims, wherein the compensation tank contains a variable amount of liquid.

8. Flow system according to any one of the preceding claims, wherein the compensation tank is set up so that the amount of liquid in it is continuously measured and the flow system is set up to regulate the circulation flow rate depending on the amount of liquid in the compensation tank.

9. Flow system according to any one of the preceding claims, wherein the compensation tank is mounted in a mass-decoupled manner.

10. Flow system according to any one of the preceding claims, further comprising a control unit which is set up to continuously receive measurement data representative of the at least one liquid parameter and to continuously receive measurement data representative of the amount of liquid in the compensation tank, and which is further set up to regulate the flow rates in the supply and discharge lines depending on the received measurement data.

11. Flow system according to any one of the preceding claims, **characterized in that** the at least one treatment liquid is acid and the flow system does not comprise a supply line for base, or that the at least one treatment liquid is base and the flow system does not comprise a supply line for acid, wherein the at least one liquid parameter is the pH value.

12. Device for the continuous treatment of a process liquid, comprising a flow system according to one of the preceding claims.

13. Device according to claim 12, further comprising a flow path arranged to receive treated process liquid, wherein the flow path is configured such that the passage of the treated process liquid through the flow path requires a predetermined minimum period of time.

14. Device according to any one of claims 12 to 13, further comprising a surge tank,
- wherein an inlet of the surge tank is connected to an outlet of an upstream unit operation,
- wherein the surge tank is set up to receive, and optionally to blend, an incoming process material,
- wherein optionally the surge tank is set up to receive a buffer,
- wherein an outlet of the surge tank is connected to the supply line for process liquid,
- wherein preferably the surge tank is set up to provide an essentially continuous outflowing liquid stream,
- wherein optionally the surge tank comprises an active mixer,
- wherein optionally the surge tank comprises means for determining a fill level of the surge tank,
- wherein optionally the surge tank comprises means for determining a liquid parameter.

15. Use of a device according to any one of claims 12 to 14 for adjusting the pH of the process liquid, for inactivating viruses in the process liquid, for solvent/detergent inactivation, for buffer conditioning, for transfection or transformation of cells, for plasmid production, for lysis of cells, for production of viral vectors, for carrying out a bio-conjugation reaction, for in vitro transcription, for precipitating DNA and/or cell fragments, for excipient addition, or for vesicle encapsulation.
